# EUROPEAN PATENT APPLICATION

(11) **EP 2 873 439 A1**
(43) Date of publication of application: **20.05.2015**
(21) Application number: 12881374.8
(22) Date of filing: 16.07.2012
(51) Int. Cl.: A61N 7/02, A61B 18/04

(54) **METHOD FOR CONTROLLING HIGH-INTENSITY FOCUSED ULTRASOUND BY USING PLURALITY OF FREQUENCIES, AND HIGH-INTENSITY FOCUSED ULTRASOUND TREATMENT APPARATUS FOR SAME**

(71) Applicant: Alpinion Medical Systems Co., Ltd., Seoul 152-848 (KR)
(72) Inventor: KIM, Dae Seung, Seoul 157-882 (KR); SON, Keonho, Seongnam-si Gyeonggi-do 463-440 (KR); KANG, Kookjin, Yongin-si Gyeonggi-do 448-536 (KR); KIM,Myungdeok, Seoul 152-050 (KR)
(74) Representative: Groth & Co. KB
(86) International application number: PCT/KR2012/005655
(87) International publication number: WO 2014/014133

(57) **Abstract**

Disclosed are a method for controlling a high-intensity focused ultrasound by using a plurality of frequencies, and a high-intensity focused ultrasound treatment apparatus for same. Provided is the high-intensity focused ultrasound treatment apparatus, comprising: a database for saving a high-intensity ultrasound table, which includes strength per ratio information and/or heat generation quantity information of a combination of a plurality of high-intensity ultrasound waves; a display portion for yielding an image; a user input portion for receiving an input of an instruction by means of user operation or input; a transrecieving portion for transmitting ultrasound waves for diagnosis to an object, and receiving an ultrasound echo signal that is reflected from the object to form a reception signal; an image processing portion for forming a B-mode image on the basis of the reception signal, and yielding the B-mode image to the display portion which is provided; a control portion for displaying through the display portion the high-intensity ultrasound table in accordance with a focus distance to the object, extracting from the database data that corresponds to selected information, which is selected through the user input portion, from the high-intensity ultrasound table, and for loading a ratio value which corresponds to the extracted data; and an ultrasound generation portion for combining the plurality of high-intensity ultrasound waves in accordance with the ratio value that is loaded and transmitting same to a specific area on the object.

## Description

### Technical Field

The present invention relates to a method for controlling high-intensity focused ultrasound by using a plurality of frequencies and a high-intensity focused ultrasound treatment apparatus therefor. More specifically, the present invention relates to a method for controlling high-intensity focused ultrasound by using a plurality of frequencies and enabling high-intensity ultrasound waves to be transmitted with a combination ratio of the plurality of frequencies according to a specific area of an object for a high-intensity focused ultrasound treatment and a high-intensity focused ultrasound treatment apparatus for the same.

### Background Art

The present discussion is only to provide background information relating to the embodiments disclosed in the application and does not constitute the prior art.

Generally, high-intensity focused ultrasound (HIFU) is used to treat (process) the biological tissue, such as cancer cells, a tumor, and a lesion. More specifically, the treatment using HIFU is a method to necrotize a biological tissue using a heat generated by transmitting a high-intensity ultrasound beam to a focused area of the biological tissue. At this time, the high-intensity ultrasound beam needs to be controlled to prevent damage to healthy tissues. High-intensity ultrasound treatment (process) can prevent surgical incision.

Traditionally, high-intensity ultrasound treatment emits ultrasound waves to obtain images of desired biological tissues that are to be treated, acquires images using a reflected echo signal resulting from the ultrasound waves, and then transmits a high-intensity ultrasound beam to the corresponding biological tissue, wherein only a single frequency is transmitted.

### Technical Problem

The purpose of the exemplary embodiments is to provide a method for controlling high-intensity focused ultrasound (HIFU) by using a plurality of frequencies to transmit high-intensity ultrasound waves for HIFU treatment to a specific area of an object with a combination ratio of the plurality of frequencies and a high-intensity focused ultrasound treatment apparatus for the same.

### Technical Solution

According to one aspect of the present invention, there is provided a high-intensity focused ultrasound (HIFU) treatment apparatus including: a database configured to store a high-intensity ultrasound table that includes information on at least one of intensities at each combination ratio of a plurality of high-intensity ultrasound waves or heat generation quantities of the same therein; a display portion configured to yield an image; a user input portion configured to receive an instruction by means of user operation or input; a transmission/reception portion configured to transmit ultrasound waves for diagnosis to an object and receive an ultrasonic echo signal that is reflected from the object to form a reception signal; an image processing portion configured to form a B-mode image on the basis of the reception signal and yield the B-mode image to the provided display portion; a control portion configured to control the high-intensity ultrasound table with respect to a focal distance to the object to be displayed through the display portion, extract from the database data that corresponds to selection information which is selected from the high-intensity ultrasound table by means of the user input portion, and load a ratio value that corresponds to the extracted data; and an ultrasound generation portion configured to combine a plurality of high-intensity ultrasound waves in accordance with the ratio value that is loaded and transmit the same to a specific area of the object.

According to another aspect of the present invention, there is provided A method for controlling high-intensity focused ultrasound by using a plurality of frequencies, the method including: a transmission and reception process for transmitting ultrasound waves for diagnosis to an object from a high-intensity focused ultrasound (HIFU) treatment apparatus and receiving an ultrasonic echo signal that is reflected from the object so as to form a reception signal; an image processing process for forming, at the HIFU treatment apparatus, a B-mode image based on the reception signal and yielding the B-mode image through a display portion provided therein; a display process for displaying the high-intensity ultrasound table with respect to a focal distance to the object through the display portion in the HIFU treatment apparatus; a control process for extracting from a database data that corresponds to selection information which is selected from the high-intensity ultrasound table by means of the user input portion in the HIFU treatment apparatus, and loading a ratio value which corresponds to the extracted data; and an ultrasound generation process for combining, at the HIFU apparatus, a plurality of high-intensity ultrasound waves in accordance with the loaded ratio value and transmitting the same to the specific area of the object.

### Advantageous Effects

As described above according to the exemplary embodiments, in the process of high-intensity focused ultrasound treatment, it may be possible to transmit high-intensity ultrasound waves in accordance with a combination ratio of a plurality of frequencies according to a specific area of an object.

In addition, according to the exemplary embodiments, by using frequency characteristics of transducers equipped in a high-intensity focused ultrasound apparatus, it may be possible to increase a cure rate of an object by simultaneously transmitting a plurality of frequencies to a specific area of an object or sequentially transmitting the plurality of frequencies in an alternating manner.

### Description of Drawings

FIG. 1 is a block diagram schematically illustrating a high-intensity focused ultrasound (HIFU) treatment apparatus according to an exemplary embodiment.
FIG. 2 is a flowchart illustrating a method for controlling high-intensity focused ultrasound by using a plurality of frequencies in accordance with an exemplary embodiment.
FIG. 3 is an illustrative diagram for explaining an ultrasound generation portion in accordance with an exemplary embodiment.
FIG. 4 is an illustrative diagram showing frequency characteristics of a transducer in accordance with an exemplary embodiment.
FIG. 5 shows illustrative diagrams showing the full width at half maximum (FWHM) according to a secondary frequency ratio in accordance with the exemplary embodiment.
FIG. 6 shows illustrative diagrams for explaining frequency intensity and heat generation quantity in accordance with the exemplary embodiment.
FIG. 7 is an illustrative diagram for explaining a ratio of channels with the secondary frequency in accordance with the exemplary embodiment.

### Mode for Invention

The invention is described more fully hereinafter with reference to the accompanying drawings, in which exemplary embodiments of the invention are shown.

High-intensity ultrasound described herein refers to ultrasound waves that are approximately 100,000 times stronger than diagnostic ultrasound. In addition, a high-intensity focused ultrasound (HIFU) treatment described herein refers to a treatment method of irradiating and focusing high-intensity ultrasound waves onto a point (a specific area) to burn the tissue of the specific area by locally heating the area to 65 °C up to 100 °C. High-intensity ultrasound waves which have an intensity that is 100,000 times stronger than the intensity of the general diagnostic ultrasound waves used for diagnosis are focused onto a point (specific area); heat is generated at the focused area, which occurs by a principle similar to that of heat generated by a convex lens that focuses the rays from the sun. The HIFU treatment is a therapeutic procedure to treat lesion inside the body without general anesthesia and without the use of needles or knives, since ultrasound waves are harmless to human body and the heat is generated only at the focus onto which the ultrasound waves are concentrated.

In addition, a B-mode described herein is an image mode that shows the movement of an object, whereby a B-mode image is in gray-scale; a C-mode image refers to a color-flow image mode; a BC-mode image shows the flow of blood or the movement of an object by using the Doppler Effect, while simultaneously providing both a B-mode image and a C-mode image to show anatomical information as well as blood flow information and object motion information. In short, the B-mode provides grey-scale images that show the movement of an object, and the C-mode provides color-flow images that show the flow of blood or the movement of an object. A high-intensity focused ultrasound (HIFU) treatment apparatus 100 described in the exemplary embodiments is capable of simultaneously providing a B-mode gray-scale image and a C-mode color-flow image, and for convenience of explanation, the embodiments will be described under the assumption that an image provided by the HIFU apparatus 100 is a B-mode image.

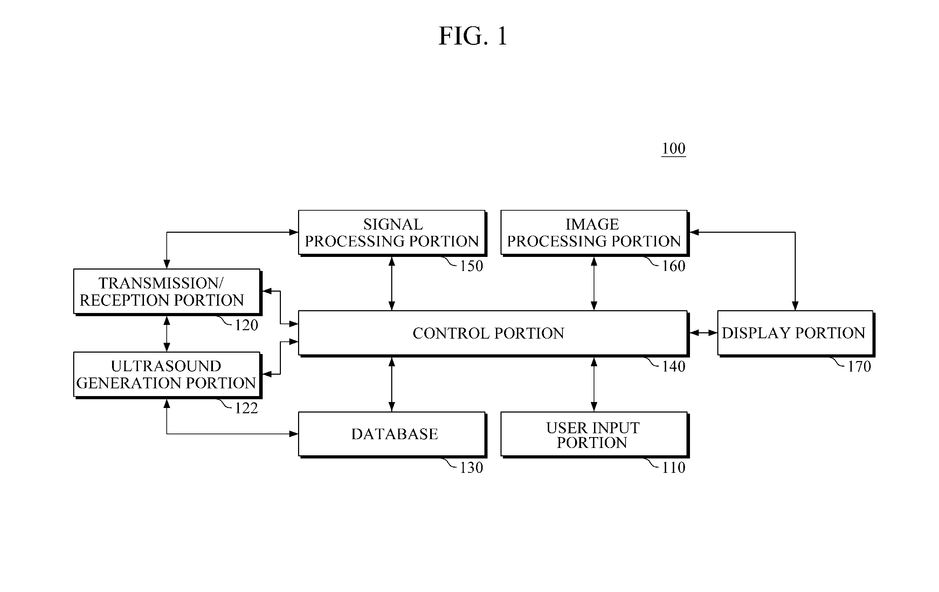

FIG. 1 is a block diagram schematically illustrating a high-intensity focused ultrasound (HIFU) treatment apparatus according to an exemplary embodiment.

The HIFU treatment apparatus 100 includes a user input portion 110, a transmission/reception portion 120, an ultrasound generation portion 122, a database 130, a control portion 140, a signal processing portion 150, an image processing portion 160, and a display portion 170. While it is described in the exemplary embodiment that the HIFU treatment apparatus 100 includes the user input portion 110, the transmission/reception portion 120, the ultrasound generation portion 122, the database 130, the control portion 140, the signal processing portion 150, the image processing portion 160, and the display portion 170, it is only to exemplify the technical idea of this embodiment and those skilled in the art to which the present embodiment pertains will appreciate that various modifications and changes are made to all elements included in the HIFU treatment apparatus 100, without departing from the essential features of the embodiment.

The user input portion 110 receives an instruction by means of user operation or input. Here, the user instruction may be a settings command to control the HIFU treatment apparatus 100.

The transmission/reception portion 120 can be operated to transmit diagnostic ultrasound waves to an object and to receive an ultrasonic echo signal reflected from the object to form a reception signal. That is, the transmission/reception portion 120 can be operated to transmit diagnostic ultrasound waves to the object to acquire a B-mode image (or a C-mode image) to and receive an ultrasonic echo signal that is reflected from the object to form the reception signal. In response to the control signal that has been received from the control portion 140, the transmission/reception portion 120 creates the reception signal based on its aforementioned process of transmitting the diagnostic ultrasound waves to the object and receiving the ultrasonic echo signal that is reflected from the object.. In addition, in response to the control signal received from the control portion 140, the transmission/reception portion 120 transmits and receives ultrasound waves at a pulse repetition frequency (PRF) within a region of interest to form the reception signal. Here, the reception signal includes a Doppler signal and a clutter signal. The Doppler signal is an ultrasound wave that is emitted from the transmission/reception portion 120 and can be reflected by the blood stream; this signal has a relatively high frequency and relatively low intensity. The clutter signal is an ultrasound wave that can be reflected by the cardiac wall or the cardiac valve; it has a relatively low frequency and relatively high intensity.

The transmission/reception portion 120 includes a probe (not shown) to transmit and receive ultrasound waves and a beamformer (not shown) to conduct transmission focusing and reception focusing of the ultrasound waves. In this case, the probe includes a plurality of 1-dimesnaional or 2-dimensional array transducers. The probe transmits focused-ultrasound beams to an object (not shown) along a transmission scanline by appropriately delaying the input time of a pulse input to each transducer. An ultrasonic echo signal reflected from the object is input to each transducer at a different reception time, and each transducer transmits the input ultrasonic echo signal to the beamformer. When the probe transmits the ultrasound waves, the beamformer adjusts a driving timing of each transducer within the probe to focus the ultrasound waves to a specific location. Furthermore, based on the fact that the ultrasonic echo signals reflected from the object reach each transducer at different times, the beamformer focuses ultrasonic echo signals by adding a time delay to each ultrasonic echo signal..

The transmission/reception portion 120 is operated basically to transmit diagnostic ultrasound waves to the object and to receive ultrasonic echo signals reflected from the object corresponding to the diagnostic ultrasound waves to form a reception signal.

The ultrasound generation portion 122 transmits high-intensity ultrasound waves to a specific area of the object. That is, the ultrasound generation portion 122 transmits the high-intensity ultrasound waves that are adjustable through the use input portion 110 to the specific area. In this case, the user primarily transmits the diagnostic ultrasound waves to the object through the transmission/reception portion 120, receives an ultrasonic echo signal that is reflected from the object, and determines the specific area of the object upon consideration of an image that has been created based on a reception signal formed from the received ultrasonic echo signal. In order to determine where the specific area is, the user may input the location value corresponding to the specific area into the user input portion 110 or by operating direction keys or a joystick. By doing so, it is possible to transmit high-intensity ultrasound waves to the specific area of the object, such as cancer tissues, tumor tissues, and lesion tissues. In such cases, the ultrasound generation portion 122 may be circular-shaped and may preferably but may not necessarily be limited to include the transmission/reception portion 120 in the center thereof.

The ultrasound generation portion, according to the exemplary embodiment, combines a plurality of high-intensity ultrasound waves in accordance with a loaded ratio value and transmits the same to a specific area of the object. More specifically, under the control of the control portion 140, the ultrasound generation portion 122 combines a plurality of high-intensity ultrasound waves according to a loaded ratio value and transmits the same to a specific area of the object. The ultrasound generation portion 122 includes primary transducers to transmit primary high-intensity ultrasound waves at a fundamental frequency to the specific area of the object and secondary transducers to transmit secondary high-intensity ultrasound waves at a secondary frequency to the same specific area of the object. Here, the primary transducers and the secondary transducers are physically the same transducer, but in the exemplary embodiments, they are separately shown for convenience of description. In addition, the ultrasound generation portion 122 includes a first group formed only by the primary transducers and a second group formed only by the secondary transducers. The ultrasound generation portion 122 is formed to have primary transducers and secondary transducers randomly arranged with each other.

The database 130 stores a reception signal received from the transmission/reception portion 120. In addition, the database 130 stores information about a plurality of cutoff frequencies used to remove a clutter signal from the reception signal. The database 130 according to the exemplary embodiment stores a high-intensity ultrasound table that includes information on at least one of the following: intensities at each combination ratio of a plurality of high-intensity ultrasound waves or heat generation quantities of the same therein. The high-intensity ultrasound table stored in the database 130 is the optimal information chosen, in accordance with the focal distance to the object, among the aforementioned two. Thus, the high-intensity ultrasound table can be updated. Here, the focal distance refers to the distance from the optical center to the primary focus. For example, it refers to the distance to a film plane in a camera for general photography, meaning the distance to a photoemissive surface of a pick-up tube in a television camera.

The control portion 140 controls the overall operation of the HIFU treatment apparatus 100. The control portion 140, in accordance with the exemplary embodiment, does the following: 1. controls the high-intensity ultrasound table with respect to the focal distance to the object, which is extracted from the database 130, to be displayed through the display portion 170, 2. extracts from the database 130 data that corresponds to selection information, which is selected from the high-intensity ultrasound table by means of the user input portion 100, 3. and loads a ratio value which corresponds to the extracted data. Thereafter, the control portion 140 controls the ultrasound generation portion 122 to combine the plurality of high-intensity ultrasound waves according to the loaded ratio value and transmit the same to the specific area of the object.

That is, when a user (operator) of the HIFU treatment apparatus 100 selects, through the user input portion 110, the information that corresponds to a ratio determined as optimal to the object from the high-intensity ultrasound table displayed on the display portion 170, the user input portion 110 transmits the selection information to the control portion 140. In this case, the selection of information from the high-intensity ultrasound table corresponding to a ratio determined as optimal to the object may be based on the user's (operator's) clinical experiences, but aspects of the present disclosure is not limited thereto, such that an algorithm for automatically selecting the information based on data previously stored in the database 170 may be employed.

Hereinafter, operation process of the control portion 140 that controls the ultrasound generation portion to combine a plurality of high-intensity ultrasound waves according to a loaded ratio value and transmit the same to a specific area of the object will be described in detail. Based on the selection information received through the user input portion 110, the control portion 140 controls the ultrasound generation portion 122 to transmit, among the plurality of high-intensity ultrasound waves, only the high-intensity ultrasound waves that are associated with a single frequency, or to transmit high-intensity ultrasound waves that are associated with two or more different frequencies. In such cases, the control portion 140 controls the ultrasound generation portion 122 to simultaneously transmit, in dual mode, high-intensity ultrasound waves associated with two or more frequencies to the object according to the selection information received through the user input portion 110. Furthermore, the control portion 140 controls the ultrasound generation portion 122 to sequentially transmit the high-intensity ultrasound waves associated with two or more frequencies to the object in an alternating manner according to the selection information received through the user input portion 110.

In addition, the operations of the control portion 140 to change the loaded ratio value in accordance with a temperature of the specific area (target area for treatment) are as follow. The control portion 140, based on the reception signal received from the transmission/reception portion 120, checks whether temperature change in the specific area belonging to the object has been detected; if the temperature change is confirmed, determines whether the changed temperature reaches a previously set target temperature; and controls the ultrasound generation portion 122 to stop transmitting high-intensity ultrasound waves based on the determination. Then, the control portion 140 controls the high-intensity ultrasound table, which relates to the focal distance to the object, to be displayed again through the display portion, extracts from the database 130 data that corresponds to selection information that has been selected from the high-intensity ultrasound table by means of the user input portion 110, and loads a ratio value that corresponds to the extracted data. Then, the control portion 140 controls the ultrasound generation portion 122 to combine a plurality of high-intensity ultrasound waves according to the loaded ratio value and to transmit the same to the specific area of the object. Moreover, the control portion 140 may control the image processing portion 160 to create at least one of a B-mode image or an elastography ultrasound image in order to measure the temperature of the specific area (target area for treatment).

Further, when receiving an input of region-of-interest (ROI) setting information from the user input portion 110, the control portion 140 may control the transmission and reception of the ultrasound waves using the input information. In addition, the control portion 140 may control ultrasound waves for B-mode images and ultrasound waves for C-mode images to be repetitively transmitted and received.

The signal processing portion 150 sets a plurality of filters with cutoff frequencies to filter out clutter signals from each pixel in a region of interest and performs clutter filtering on the reception signals from the transmission/reception portion 120. The signal processing portion 150 may perform signal processing, such as gain control for image optimization, on the reception signal from the transmission/reception portion 120. Furthermore, the signal processing portion 150 performs low bandpass filtering on the reception signal and transmits the same to the image processing portion 160.

The image processing portion 160 can be operated to create a B-mode image, a C-mode image, or an elastography ultrasound image, and yield the same through the display portion 170. In an elastography ultrasound image, stiffer tissues represented by a darker color and softer tissues by a brighter color, which are acquired by pressurizing an object and observing the difference in the before and after images of the tissues. Basically, the image processing portion 160 can be operated to create a B-mode image based on the reception signal and yield the same through the display portion 170.

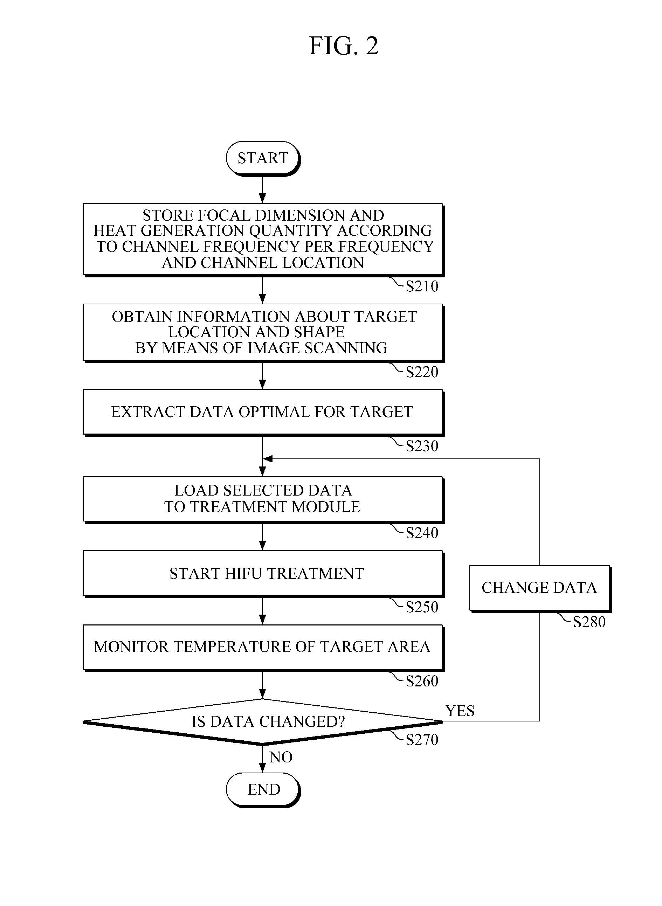

FIG. 2 is a flowchart illustrating a method for controlling high-intensity focused ultrasound by using a plurality of frequencies in accordance with an exemplary embodiment.

A database 130 included in the HIFU treatment apparatus 100 stores a high-intensity ultrasound table that includes information on at least one of about the following: information about intensities at each combination ratio of a plurality of high-intensity ultrasound waves or information about heat generation quantities of the same therein in S210. Here, the high-intensity ultrasound table to be stored in the database 130 is the optimal information chosen, in accordance with the focal distance to the object, among the aforementioned two. Thus, the high-intensity ultrasound table can be updated.

A transmission/reception portion 120 included in the HIFU treatment apparatus 100 transmits diagnostic ultrasound waves to the object and receives an ultrasonic echo signal which is reflected from the object to form a reception signal in S220. In S230, a control portion 140 included in the HIFU treatment apparatus 100 does the following: 1. controls a high-intensity ultrasound table with respect to a focal distance to the object, which is extracted from the database 130, to then be displayed through the display portion 170, 2. and extracts from the database 130 data that corresponds to selection information which has been selected from the high-intensity ultrasound table by means of a user input portion 110.

The control portion 140 provided within the HIFU treatment apparatus 100 loads a ratio value that corresponds to the extracted data in S240. The control portion 140 of the HIFU treatment apparatus 100 controls an ultrasound generation portion 122 to combine a plurality of high-intensity ultrasound waves in accordance with the loaded ratio value and transmit the same to a specific area of the object in S250. More specifically, in S250, based on the selection information received through the user input portion 110, the HIFU treatment apparatus 100 controls the ultrasound generation portion 122 to transmit, among the plurality of high-intensity ultrasound waves, only the high-intensity ultrasound waves that are associated with a single frequency, or to transmit high-intensity ultrasound waves that are associated with two or more different frequencies. In this case, the control portion 140 controls the ultrasound generation portion 122 to simultaneously transmit, in dual mode, high-intensity ultrasound waves associated with two or more frequencies to the object according to the selection information received through the user input portion 110. Furthermore, the control portion 140 controls the ultrasound generation portion 122 to sequentially transmit the high-intensity ultrasound waves associated with two or more frequencies to the object in an alternating manner according to the selection information received through the user input portion 110.

The control portion 140 of the HIFU treatment apparatus 100, based on the reception signal received from the transmission/reception portion 120, checks whether temperature change in the specific area belonging to the object has been detected; if the temperature change is confirmed as a result of checking, determines whether the changed temperature reaches a previously set target temperature; and controls the ultrasound generation portion 122 to stop transmitting high-intensity ultrasound waves on the basis of the determination in S260.

The control portion 140 of the HIFU treatment apparatus 100 controls the high-intensity ultrasound table, which relates to the focal distance (focal depth) to the object, to be displayed again, and checks whether selection information among the high-intensity ultrasound table is input through the user input portion in S270. In response to the determination in S270 that the selection information is input through the user input portion 110, the control portion 140 of the HIFU treatment apparatus 100 extracts associated data from the database 130 in S280. After S280, the HIFU treatment apparatus 100 repeatedly performs operations S240 through S270.

While it is described in FIG. 2 that operations S210 to S280 are sequentially performed, it is only to exemplify the technical idea of this embodiment, and those skilled in the art to which the present embodiment pertains will appreciate that various modifications and changes are made to the method shown in FIG. 2 in such a manner that the sequence shown in FIG. 2 is changed or at least two of operations S210 to S280 are performed concurrently, and thus FIG. 2 is not limited in time-series order.

As described above, the method for controlling high-intensity focused ultrasound in accordance with the exemplary embodiment shown in FIG. 2 may be implemented as a program and recorded in a computer-readable recording medium. The computer-readable recording medium, in which the program for implementing the method for correcting errors in 3D images in accordance with an embodiment of the present invention is recorded, comprises all types of recording devices in which data readable by a computer system is stored. Examples of the computer-readable recording medium may include ROMs, RAMS, CD-ROMs, magnetic tape, floppy discs, optical data storage devices, etc. Moreover, the computer-readable recording medium may be implemented in the form of carrier wave (e.g., transmission through the Internet). Furthermore, the computer-readable recording media are distributed in computer systems connected through the network such that a computer-readable code can be stored and executed in a distributed manner. In addition, functional programs, code, and code segments for implementing the present embodiment can be easily construed by programmers skilled in the art to which the present embodiment pertains.

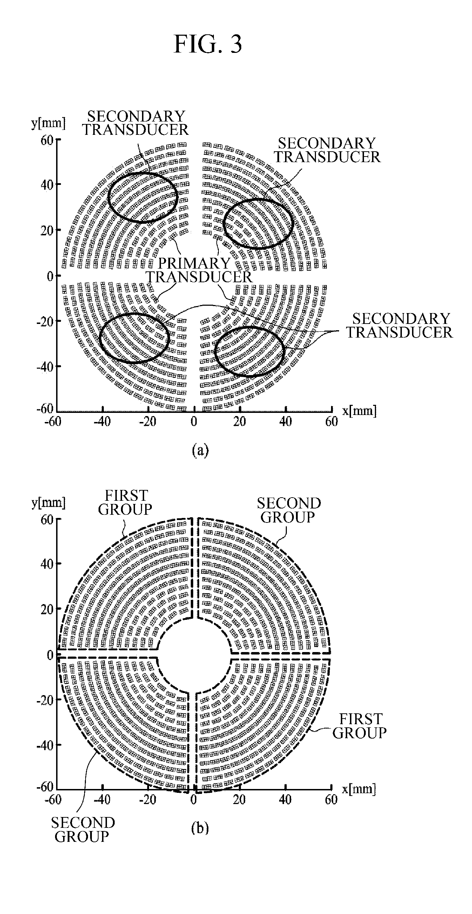

FIG. 3 is an illustrative diagram for explaining an ultrasound generation portion in accordance with an exemplary embodiment.

The ultrasound generation portion 122 transmits high-intensity ultrasound waves to a specific area of a target object using equipped transducers. As shown in FIG. 3, the ultrasound generation portion 122 may have designated groups or have random arrangement according to the fundamental high-intensity ultrasound and the second high-intensity ultrasound to be transmitted to the object. The ultrasound generation portion 122 combines a plurality of high-intensity ultrasound waves according to a loaded ratio value and transmits the same to the specific area of the object.

The ultrasound generation portion 122 transmits high-intensity ultrasound waves to a specific location that has been adjusted through the user input portion 110. Here, a user (operator) first transmits diagnostic ultrasound waves to the object through the transmission/reception portion 120, receives an ultrasonic echo signal reflected from the object to form a reception signal, and determines the specific area of the object from an image generated based on the reception signal. In this case, the user may determine the specific area by inputting a location value corresponding to the specific area to the user input portion 110 or by operating direction keys, such as a joystick. By doing so, it is possible to transmit high-intensity ultrasound waves to the specific area of the object, such as a cancer tissue, a tumor tissue, and a lesion tissue. In this case, the ultrasound generation portion 122 may be circular-shaped as shown in FIG. 3, and preferably but may not necessarily be limited to include the transmission/reception portion 120 in the center thereof.

That is, under the control of the control portion 140, the ultrasound generation portion 122 combines a plurality of high-intensity ultrasound waves according to the loaded ratio value and transmits the same to the specific area of the object. The ultrasound generation portion 122 includes, as shown in FIG. 3, primary transducers to transmit a primary high-intensity ultrasound wave at a fundamental frequency to the specific area of the object and secondary transducers to transmit a second high-intensity ultrasound wave at a secondary frequency to the same specific area of the object. Here, the primary transducers and the secondary transducers are physically the same transducer, but in the exemplary embodiments, they are separately shown for convenience of description.

More specifically, as shown in FIG. 3 (b), the ultrasound generation portion 122 includes a first group formed only by the primary transducers and a second group formed only by the secondary transducers. Moreover, as shown in FIG. 3 (a), the ultrasound generation portion 122 may be formed to have primary transducers and secondary transducers randomly arranged with each other.

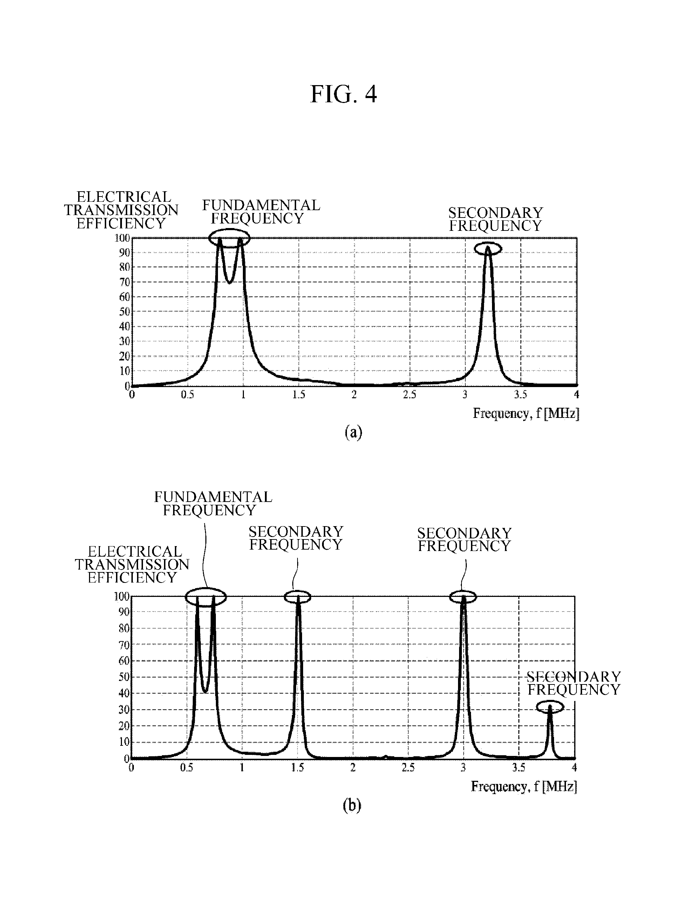

FIG. 4 is an illustrative diagram showing frequency characteristics of a transducer in accordance with an exemplary embodiment.

The ultrasound generation portion 122 includes transducers to transmit high-intensity ultrasound waves. In this case, each transducer converts an electrical analog signal into high-intensity ultrasound and emits the same to a target object. Generally, the transducer is formed by a plurality of transducer elements coupled therewith.

In a case where the transducers equipped in the ultrasound generation portion 122 have frequency characteristics as shown in FIG. 4(a), the fundamental frequency ranges between about 0.75 and 0.9 MHz, and the secondary frequency is about 3.2 MHz. It is only to exemplify the technical idea of this embodiment and those skilled in the art to which the present embodiment pertains will appreciate that various modifications and changes are made to the frequencies, without departing from the frequency characteristics of the transducers.

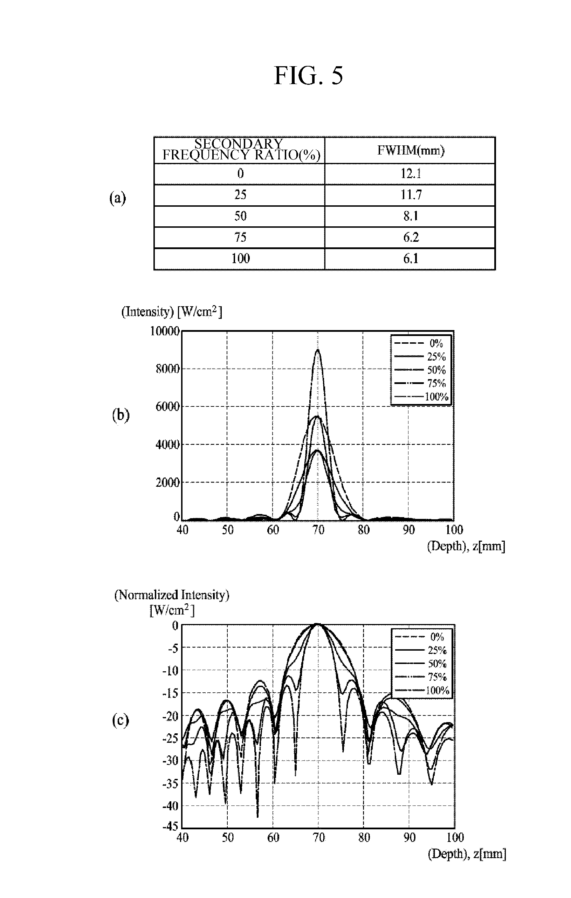

FIG. 5 shows illustrative diagrams showing a full width at half maximum (FWHM) according to a secondary frequency ratio in accordance with the exemplary embodiment.

FIG. 5 is an illustrative diagram showing bandwidth changes along an axial direction in accordance with a ratio of channels with the secondary frequency when the ultrasound generation portion 122 transmits the fundamental frequency and the secondary frequency. As shown in FIG. 5(a), a secondary frequency ratio may be set to 0 %, 25 %, 50 %, 75 %, and 100 %, and FWHM for each ratio may be set to 12.1, 11.7, 8.1, 6.2 and 6.1, respectively. Here, the FWHM refers to a spectral width at the half of a peak on a spectrum, such as frequency response.

Referring to FIG. 5(b), in a case where the depth to the object is about 70 mm, it is seen that the intensity is about 9000 W/cm² at the secondary frequency ratio of 0 %; the intensity is about 5800 W/cm² at the secondary frequency ratio of 25 %; the intensity is about 5800 W/cm² at the secondary frequency ratio of 50 %; the intensity is about 3800 W/cm² at the secondary frequency ratio of 75 %; and the intensity is about 3800 W/cm² at the secondary frequency ratio of 100 %. Referring to FIG. 5(c), the average intensity varies with depth.

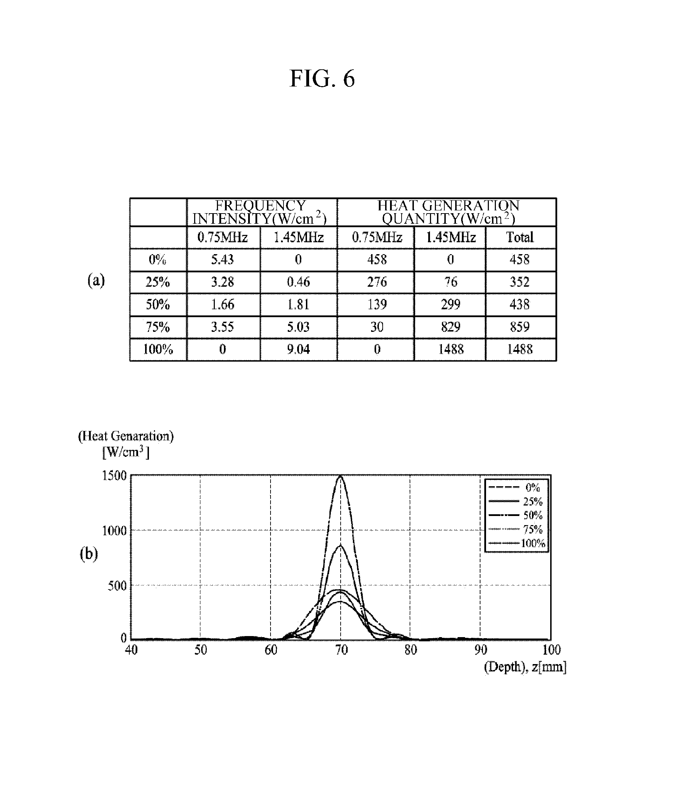

FIG. 6 shows illustrative diagrams for explaining frequency intensity and heat generation quantity in accordance with the exemplary embodiment.

FIG. 6 shows illustrative diagrams showing heat generation quantity changes in accordance with the ratio of channels with the secondary frequency when the ultrasound generation portion 122 transmits the fundamental frequency and the secondary frequency. As shown in FIG. 6(a), assuming that the fundamental frequency is 0.75 MHz and the secondary frequency is 1.45 MHz, in a case where the secondary frequency ratio is about 0 %, the intensity is about 5.43 W/cm² at the fundamental frequency (0.75 MHz) and about 0 W/cm² at the secondary frequency (1.45 MHz). In this case, the heat generation quantity is about 458 W/cm³ at the fundamental frequency (0.75 MHz) while it is 0 W/cm³ at the secondary frequency (1.45 MHz), thus resulting in the total heat generation quantity of about 458 W/cm³.

In the case of the secondary frequency ratio of about 25 %, the intensity is 3.28 W/cm² at the fundamental frequency (0.75 MHz) and it is 0.46 W/cm² at the secondary frequency (1.45 MHz). At this time, the heat generation quantity is about 276 W/cm³ at the fundamental frequency (0.75 MHz) while it is about 76 W/cm³ at the secondary frequency (0.75 MHz), thus resulting in the total heat generation quantity of about 352 W/cm³. In the case of the secondary frequency ratio of about 50 %, the intensity is about 1.66 W/cm² at the fundamental frequency (0.75 MHz) while it is about 1.81 W/cm² at the secondary frequency (1.45 MHz). At this time, the heat generation quantity is about 139 W/cm³ at the fundamental frequency (0.75 MHz) while it is about 299 W/cm³ at the secondary frequency (1.45 MHz), thus resulting in the total heat generation quantity of about 438 W/cm³. In the case of the secondary frequency ratio of about 75 %, the intensity is about 3.55 W/cm² at the fundamental frequency (0.75 MHz) while it is about 5.03 W/cm² at the secondary frequency (1.45 MHz). At this time, the heat generation quantity is about 30 W/cm³ at the fundamental frequency (0.75 MHz) while it is about 829 W/cm³ at the secondary frequency (1.45 MHz), thus resulting in the total heat generation quantity of about 859 W/cm³. In the case of the secondary frequency ratio of about 100 %, the intensity is about 0 W/cm² at the fundamental frequency (0.75 MHz) while it is about 9.04 W/cm² at the secondary frequency (1.45 MHz). At this time, the heat generation quantity is about 0 W/cm³ at the fundamental frequency (0.75 MHz) while it is about 1488 W/cm³ at the secondary frequency (1.45 MHz), thus resulting in the total heat generation quantity of about 1488 W/cm³.

Referring to FIG. 6(b), in a case where the depth to the object is about 70 mm, it is seen that the heat generation quantity is about 460 W/cm³ at the secondary frequency ratio of 0 %; the heat generation quantity is about 350 W/cm³ at the secondary frequency ratio of 25 %; the heat generation quantity is about 440 W/cm³ at the secondary frequency ratio of 50 %; the heat generation quantity is about 860 W/cm² at the secondary frequency ratio of 75 %; and the heat generation quantity is about 1490 W/cm³ at the secondary frequency ratio of 100 %.

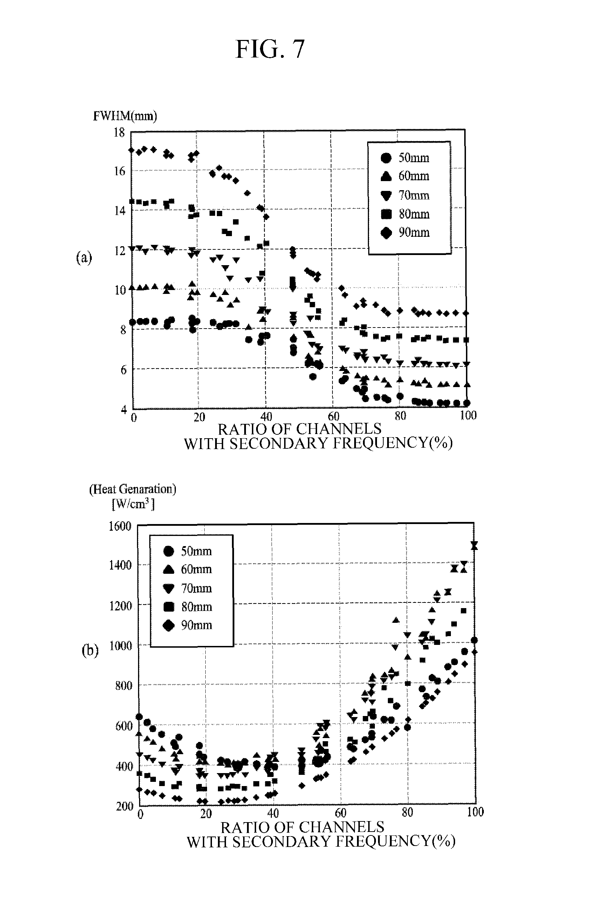

FIG. 7 is an illustrative diagram for explaining a ratio of channels with the secondary frequency in accordance with the exemplary embodiment.

As shown in FIG. 7(a), it is seen that, in the case where the depth to the object is 50 mm, a value of FWHM ranges between 4 mm and 8 mm according to the ratio of channels with the secondary frequency; in the case where the depth to the object is 60 mm, a value of FWHM ranges between 5 mm and 10 mm according to the ratio of channels with the secondary frequency; in the case where the depth to the object is 70 mm, a value of FWHM ranges between 6 mm and 12 mm according to the ratio of channels with the secondary frequency; in the case where the depth to the object is 80 mm, a value of FWHM ranges between 7 mm and 15 mm according to the ratio of channels with the secondary frequency; and in the case where the depth to the object is 90 mm, a value of FWHM ranges between 8 mm and 17 mm according to the ratio of channels with the secondary frequency.

As shown in FIG. 7(b), it is seen that, in the case where the depth to the object is 50 mm, a value of heat generation quantity ranges between 650 W/cm³ and 100 W/cm³ according to the ratio of channels with the secondary frequency; in the case where the depth to the object is 60 mm, a value of heat generation quantity ranges between 580 W/cm³ and 1250 W/cm³ according to the ratio of channels with the secondary frequency; in the case where the depth to the object is 70 mm, a value of heat generation quantity ranges between 430 W/cm³ and 1500 W/cm³ according to the ratio of channels with the secondary frequency; in the case where the depth to the object is 80 mm, a value of heat generation quantity ranges between 380 W/cm³ and 120 W/cm³ according to the ratio of channels with the secondary frequency; and in the case where the depth to the object is 90 mm, a value of heat generation quantity ranges between 300 W/cm³ and 1000 W/cm³ according to the ratio of channels with the secondary frequency.

The above description is only to exemplify the technical idea of the embodiments, and it is apparent to those skilled in the art that various modifications and changes can be made to the embodiments without departing from the essential features of the embodiments. The exemplary embodiments should be considered in descriptive sense only and not for purposes of limitation. Therefore, the scope of the embodiments is defined not by the detailed description of the disclosure but by the appended claims, and all differences within the scope will be construed as being included in the present embodiments.

### <Explanation of Reference Numerals>

100: high-intensity focused ultrasound treatment apparatus
110: user input portion 120: transmission/reception portion
122: ultrasound generation portion 130: database
140: control portion 150: signal processing portion
160: image processing portion 170: display portion

## Claims

1. A high-intensity focused ultrasound (HIFU) treatment apparatus comprising:
a database configured to store a high-intensity ultrasound table that includes information on at least one of intensities at each combination ratio of a plurality of high-intensity ultrasound waves or heat generation quantities of the same therein;
a display portion configured to yield an image;
a user input portion configured to receive an instruction by means of user operation or input;
a transmission/reception portion configured to transmit ultrasound waves for diagnosis to an object and receive an ultrasonic echo signal that is reflected from the object to form a reception signal;
an image processing portion configured to form a B-mode image on the basis of the reception signal and yield the B-mode image to the provided display portion;
a control portion configured to control the high-intensity ultrasound table with respect to a focal distance to the object to be displayed through the display portion, extract from the database data that corresponds to selection information which is selected from the high-intensity ultrasound table by means of the user input portion, and load a ratio value that corresponds to the extracted data; and
an ultrasound generation portion configured to combine a plurality of high-intensity ultrasound waves in accordance with the ratio value that is loaded and transmit the same to a specific area of the object.

2. The HIFU treatment apparatus of claim 1, wherein the control portion controls, based on the selection information, the ultrasound generation portion to transmit, among the plurality of high-intensity ultrasound waves, only the high-intensity ultrasound waves that are associated with a single frequency, or to transmit high-intensity ultrasound waves that are associated with two or more different frequencies.

3. The HIFU treatment apparatus of claim 2, wherein the control portion controls the ultrasound generation portion to simultaneously transmit, in dual mode, high-intensity ultrasound waves associated with two or more frequencies to the object according to the selection information.

4. The HIFU treatment apparatus of claim 2, wherein the control portion controls the ultrasound generation portion to sequentially transmit the high-intensity ultrasound waves associated with two or more frequencies to the object in an alternating manner according to the selection information.

5. The HIFU treatment apparatus of claim 1, wherein the ultrasound generation portion comprises primary transducers (HIFU transducers) to transmit primary high-intensity ultrasound waves at a fundamental frequency to the specific area of the object and secondary transducers to transmit second high-intensity ultrasound waves at a secondary frequency to the same specific area of the object.

6. The HIFU treatment apparatus of claim 1, wherein the ultrasound generation portion comprises a first group formed only by the primary transducers and a second group formed only by the secondary transducers.

7. The HIFU treatment apparatus of claim 5, wherein the ultrasound generation portion is formed to have the primary transducers and the secondary transducers randomly arranged with each other.

8. The HIFU treatment apparatus of claim 1, wherein the control portion checks, based on the reception signal, whether temperature change in the specific area belonging to the object has been detected; if the temperature change is confirmed as a result of checking, determines whether the changed temperature reaches a previously set target temperature; controls the ultrasound generation portion to stop transmitting high-intensity ultrasound waves on the basis of the determination; subsequently, controls the high-intensity ultrasound table, which relates to the focal distance to the object, to be displayed again through the display portion; extracts from the database the data that corresponds to the selection information which is selected from the high-intensity ultrasound table by means of the user input portion; and loads a ratio value that corresponds to the extracted data.

9. A method for controlling high-intensity focused ultrasound by using a plurality of frequencies, the method comprising:
a transmission and reception process for transmitting ultrasound waves for diagnosis to an object from a high-intensity focused ultrasound (HIFU) treatment apparatus and receiving an ultrasonic echo signal that is reflected from the object so as to form a reception signal;
an image processing process for forming, at the HIFU treatment apparatus, a B-mode image based on the reception signal and yielding the B-mode image through a display portion provided therein;
a display process for displaying the high-intensity ultrasound table with respect to a focal distance to the object through the display portion in the HIFU treatment apparatus;
a control process for extracting from a database data that corresponds to selection information which is selected from the high-intensity ultrasound table by means of the user input portion in the HIFU treatment apparatus, and loading a ratio value which corresponds to the extracted data; and
an ultrasound generation process for combining, at the HIFU apparatus, a plurality of high-intensity ultrasound waves in accordance with the loaded ratio value and transmitting the same to the specific area of the object.

10. The method of claim 9, wherein the control process comprises a process for controlling, based on the selection information, the ultrasound generation portion to transmit, among the plurality of high-intensity ultrasound waves, only the high-intensity ultrasound waves that are associated with a single frequency, or transmit high-intensity ultrasound waves that are associated with two or more different frequencies.

11. The method of claim 10, wherein the control process comprises a process for controlling the ultrasound generation portion to simultaneously transmit, in dual mode, high-intensity ultrasound waves associated with two or more frequencies to the object according to the selection information.

12. The method of claim 10, wherein the control process comprises a process for controlling the ultrasound generation portion to sequentially transmit the high-intensity ultrasound waves associated with two or more frequencies to the object in an alternating manner according to the selection information.
